(19) Europäisches Patentamt
European Patent Office
Office européen des brevets

(11) Veröffentlichungsnummer: **0 010 288**
**B1**

# (12) EUROPÄISCHE PATENTSCHRIFT

(45) Veröffentlichungstag der Patentschrift: **25.11.81**

(51) Int. Cl.³: **C 07 D 307/60**

(21) Anmeldenummer: **79103978.7**

(22) Anmeldetag: **15.10.79**

## (54) Verfahren zur Herstellung von Tetronsäuremethylester.

(30) Priorität: **16.10.78 DE 2845037**

(43) Veröffentlichungstag der Anmeldung: **30.04.80 Patentblatt 80/9**

(45) Bekanntmachung des Hinweises auf die Patenterteilung: **25.11.81 Patentblatt 81/47**

(84) Benannte Vertragsstaaten: **AT BE CH FR GB IT LU NL SE**

(56) Entgegenhaltungen:
**DE-A-2 116 416**
**DE-A1-2 707 104**
**DE-A1-2 731 483**
**DE-B2-2 154 439**
**DE-B2-2 509 576**

(73) Patentinhaber: **Dr. Willmar Schwabe, Willmar-Schwabe-Strasse 4, D-7500 Karlsruhe-Durlach (DE)**

(72) Erfinder: **Pelter, Andrew, Dr., 189 Mayals Road, Mayals, Swansea (GB)**

(74) Vertreter: **VOSSIUS VOSSIUS TAUCHNER HEUNEMANN RAUH, Siebertstrasse 4 P.O. Box 86 07 67, D-8000 München 86 (DE)**

EP 0 010 288 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von Tetronsäuremethylester

Tetronsäuremethylester, auch als 3-Methoxybut-3-enolid bezeichnet, der Formel

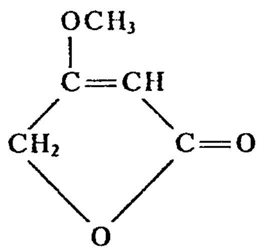

ist eine bekannte Verbindung, die ein wertvolles organisches Zwischenprodukt darstellt. Die Verbindung wurde bisher durch Umsetzung von 3-Hydroxybut-3-enolid (Tetronsäure) mit Diazomethan hergestellt; vgl. C.T. Calam, A.R. Todd und W.S. Waring, Biochem. J., Bd. 45 (1949), S. 520. Das eingesetzte 3-Hydroxybut-3-enolid wird ausgehend von Acetessigsäureäthylester in einem mehrstufigen Verfahren hergestellt. Das Gesamtverfahren kann durch folgendes Reaktionsschema wiedergegeben werden:

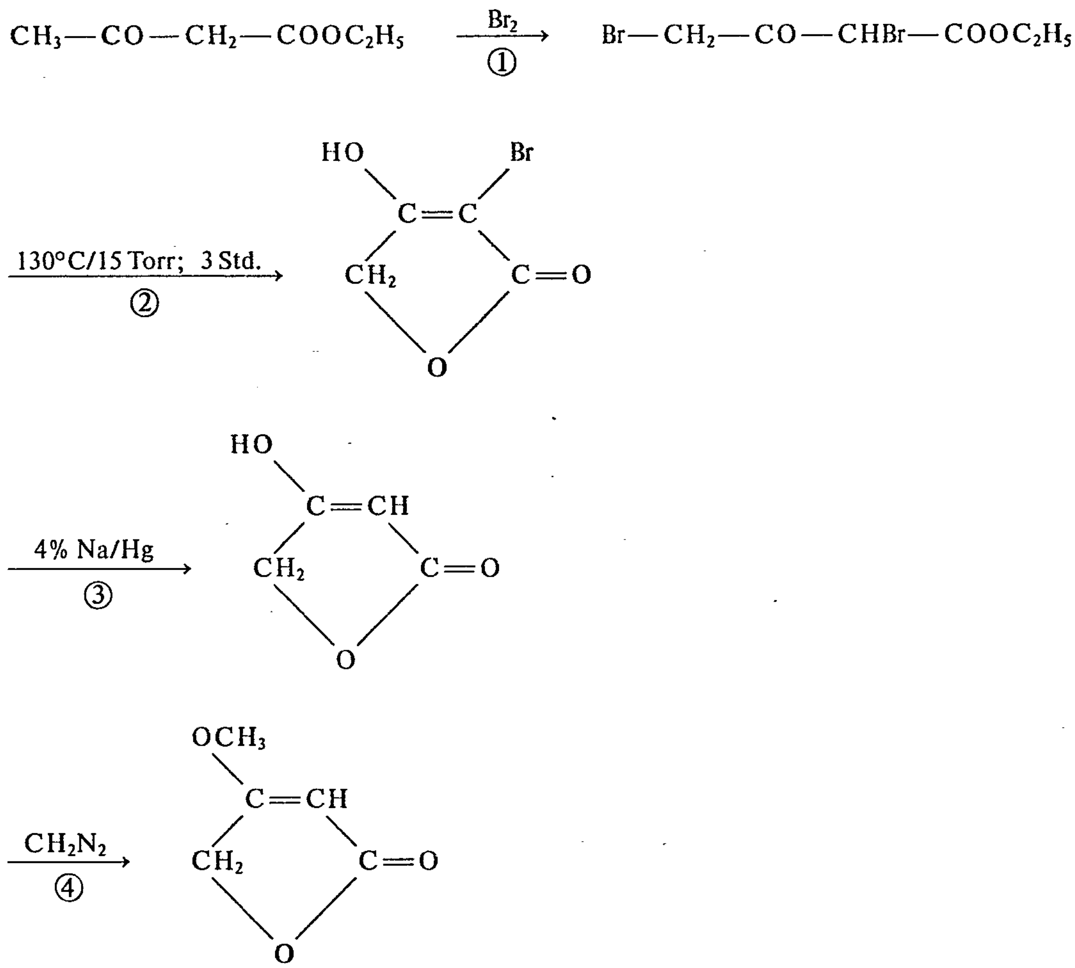


Die Stufen 1 bis 3 sind von W.D. Kumler, J. Am. Chem. Soc., Bd. 60 (1938), S. 859, beschrieben. Die Gesamtausbeute dieser 3 Stufen beträgt nur etwa 36%. Die Methylierung mit Diazomethan verläuft vermutlich in guter Ausbeute, doch sind keine Zahlen angegeben.

Das bekannte Verfahren zur Herstellung von Tetronsäuremethylester ist abgesehen von seiner niedrigen Gesamtausbeute aus ersichtlichen Gründen in technischem Maßstab schwer durchführbar. Insbesondere ist die Umsetzung mit Diazomethan aufwendig und gefährlich.

Der Erfindung liegt die Aufgabe zugrunde, ein leistungsfähiges neues Verfahren zur Herstellung des Tetronsäuremethylesters aus leicht zugänglichen Ausgangsverbindungen zu schaffen, das sich auch für die technische Herstellung der Verbindung eignet. Die Lösung dieser Aufgabe beruht auf dem Befund, daß sich 4-Brom-3-methoxybut-2-encarbonsäure-nieder-alkylester, wie der technisch leicht

zugängliche Äthylester (vgl. GB-PS 1 201 628), in guter Ausbeute in Tetronsäuremethylester überführen lassen.

Gegenstand der Erfindung ist somit ein Verfahren zur Herstellung von Tetronsäuremethylester, das dadurch gekennzeichnet ist, daß man einen 4-Brom-3-methoxybut-2-encarbonsäure-nieder-alkylester mit einer Lewis-Säure in einem organischen Lösungsmittel erhitzt.

Als Ausgangsverbindungen werden im erfindungsgemäßen Verfahren wegen ihrer leichten Zugänglichkeit die niederen Alkylester der 4-Brom-3-methoxybut-2-encarbonsäure, beispielsweise der Methyl- oder Äthylester, eingesetzt.

Der im erfindungsgemäßen Verfahren bevorzugt eingesetzte 4-Brom-3-methoxybut-2-encarbonsäureäthylester wird nach folgendem Reaktionsschema hergestellt:

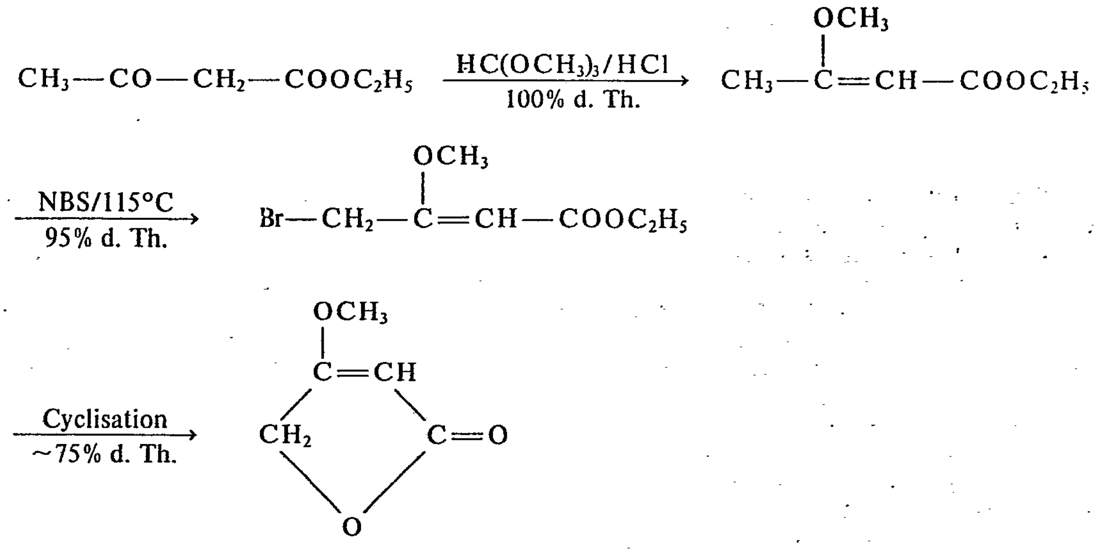


Es ist ersichtlich, daß die Gesamtausbeute des Verfahrens, ausgehend von Acetessigsäureäthylester etwa 71% d. Th. beträgt, während sie bei dem vorstehend beschriebenen bekannten Verfahren höchstens 18% beträgt.

Die Herstellung des $\beta$-Methoxycrotonsäureäthylesters erfolgt nach dem in der GB-PS 1 137 466 und in J. Org. Chem., Bd. 29 (1964), S. 3161, beschriebenen Verfahren. Die Bromierung mit Bromsuccinimid (NBS) ist in der GB-PS 1 201 628 beschrieben.

Als Lewis-Säuren kommen im erfindungsgemäßen Verfahren vorzugsweise wasserfreie Metallhalogenide, insbesondere Zinkbromid, Zinkchlorid, Aluminiumtrichlorid, Aluminiumtribromid und Zinntetrachlorid, in Frage. Ferner kann mit gutem Erfolg auch Borfluorid-ätherat verwendet werden. Der Katalysator kann in Mengen von etwa 0,01 bis 0,1 Mol pro Mol 4-Brom-3-methoxybut-2-en-carbonsäureester verwendet werden. Die optimale Menge läßt sich durch einige Vorversuche leicht bestimmen.

Als organische Lösungsmittel werden zweckmäßig solche Lösungsmittel verwendet, die durch die Lewis-Säure nicht zersetzt werden. Geeignete Lösungsmittel sind z. B. aliphatische und aromatische Kohlenwasserstoffe, wie Petroläther, Ligroin, Benzol, Toluol, Xylol, Nitrobenzol und Schwefelkohlenstoff.

Die Reaktionstemperatur hängt von der Art des verwendeten Lösungsmittels ab. Im allgemeinen wird die Umsetzung bei der Rückflußtemperatur des verwendeten Lösungsmittels durchgeführt. Die Reaktionszeit hängt von der Reaktionstemperatur ab; im allgemeinen beträgt sie 5 bis 14 Stunden.

Tetronsäuremethylester ist ein wertvolles chemisches Zwischenprodukt, das mit reaktionsfähigen Carbonylverbindungen, wie Aldehyden oder Säurechloriden, zu Verbindungen mit wertvollen pharmakologischen Eigenschaften kondensiert werden kann. Im Fall von Aldehyden können 1 oder 2 Mol Aldehyd mit 1 Mol Tetronsäuremethylester in Form dessen Lithiumsalzes zu den entsprechenden Produkten umgesetzt werden. Die Kondensationsreaktion erfolgt an der Methylengruppe in der 4-Stellung.

Das Lithiumsalz kann durch Umsetzung von Tetronsäuremethylester bei −78°C in Tetrahydrofuran mit n-Butyllithium wie folgt hergestellt werden:

### Verfahren A

200 ml wasserfreies Tetrahydrofuran (THF) und 7 ml (50 mMol) frisch destilliertes wasserfreies Diisopropylamin werden mittels einer Injektionsspritze in einen mit einem Gummistopfen verschlossenen Zweihalskolben injiziert, der mit wasserfreiem Stickstoff gespült worden ist und in dem eine Stickstoffatmosphäre als Schutzgas aufrechterhalten wird. Sodann wird in gleicher Weise

bei −78°C eine Lösung von n-Butyllithium in Heptan (25 ml einer 2,0molaren Lösung) in den Zweihalskolben unter Rühren injiziert. Nach 15minütigem Rühren werden 7,5 ml Hexamethylphosphorsäuretriamid (HMPT) zugegeben, und das Gemisch wird weitere 15 Minuten bei −78°C gerührt. Sodann wird mittels der Injektionsspritze eine Lösung von 5,7 g (50 mMol) Tetronsäuremethylester in 100 ml wasserfreies THF injiziert und das Gemisch weitere 10 Minuten bei −78°C gerührt. Die erhaltene Lösung des 5-Lithiumsalzes des Tetronsäuremethylesters kann unmittelbar für die weitere Umsetzung mit Aldehyden oder Säurechloriden verwendet werden.

Verfahren B

Das Verfahren A wird wiederholt, jedoch wird kein HMPT zugesetzt. Es wird das gleiche Ergebnis erhalten.

Beispiel

100 g 4-Brom-3-methoxybut-2-encarbonsäureäthylester, 0,5 g wasserfreies Zinkbromid und 100 ml wasserfreies p-Xylol werden in einem 500 ml fassenden Rundkolben vorgelegt, der mit einem Rückflußkühler ausgerüstet ist. Das Gemisch wird 6 Stunden unter Rückfluß erhitzt. Danach wird das Lösungsmittel unter vermindertem Druck abdestilliert. Der Rückstand wird mit 300 ml eines 2 : 8-Gemisches von Chloroform und Diäthyläther versetzt. Dabei fällt das Zinkbromid als Schmiere aus. Die überstehende Lösung wird dekantiert und 18 Stunden bei 0°C stehengelassen. Die entstandenen Kristalle des Tetronsäuremethylesters werden abfiltriert und getrocknet. Ausbeute 30 g vom F. 64 bis 65°C. Das Filtrat wird unter vermindertem Druck eingedampft und der Rückstand mit 75 ml des 2 : 8-Gemisches von Chloroform und Diäthyläther versetzt. Nach weiterem Stehenlassen in der Kälte werden noch 5 g Produkt vom F. 64 bis 65°C isoliert. Das Filtrat wird nochmals in gleicher Weise behandelt. Es werden weitere 2 g Produkt vom F. 64 bis 65°C erhalten. Die Gesamtausbeute beträgt 37 g (75% d. Th.).

In analoger Weise wird 4-Brom-3-methoxybut-2-encarbonsäureäthylester mit äquivalenten Mengen wasserfreiem Zinkchlorid, Aluminiumtrichlorid, Aluminiumtribromid, Zinntetrachlorid oder Bortrifluorid-ätherat umgesetzt. Es wird in jedem Fall der Tetronsäuremethylester in Ausbeuten zwischen 70 und 80% der Theorie erhalten.

Gleiche Ergebnisse werden erhalten bei Verwendung von 4-Brom-3-methoxybut-2-encarbonsäuremethylester.

**Patentansprüche**

1. Verfahren zur Herstellung von Tetronsäuremethylester, dadurch gekennzeichnet, daß man einen 4-Brom-3-methoxybut-2-encarbonsäure-nieder-alkylester mit einer Lewis-Säure als Katalysator in einem organischen Lösungsmittel erhitzt.

2. Verfahren nach Anspruch 1, dadurch gekennzeichnet, daß man als Lewis-Säure wasserfreies Zinkbromid, Zinkchlorid, Aluminiumtrichlorid, Aluminiumtribromid, Zinntetrachlorid oder Borfluorid-ätherat verwendet.

3. Verfahren nach Anspruch 1, dadurch gekennzeichnet, daß man 4-Brom-3-methoxybut-2-encarbonsäureäthylester einsetzt.

**Claims**

1. Process for preparing methyl tetronate, characterized in that a lower alkyl 4-bromo-3-methoxybut-2-encarboxylate is heated in an organic solvent with a Lewis acid as catalyst.

2. Process according to claim 1, characterized in that as Lewis acid anhydrous zinc bromide, zinc chloride, aluminium trichloride, aluminium tribromide, tin tetrachloride or boron fluoride-etherate is used.

3. Process according to claim 1, characterized in that ethyl 4-bromo-3-methoxybut-2-encarboxylate is used.

**Revendications**

1. Procédé de préparation d'ester méthylique d'acide tétronique, caractérisé en ce qu'on chauffe un ester alkylique inférieur d'acide 4-bromo-3-méthoxybut-2-ènecarboxylique avec un acide de Lewis comme catalyseur, dans un solvant organique.

2. Procédé suivant la revendication 1, caractérisé en ce que, comme acide de Lewis, on utilise du bromure de zinc, du chlorure de zinc, du trichlorure d'aluminium, du tribromure d'aluminium, du tétrachlorure d'étain ou de l'éthérate de fluorure de bore anhydre.

3. Procédé suivant la revendication 1, caractérisé en ce qu'on met en oeuvre de l'ester éthylique d'acide 4-bromo-3-méthoxybut-2-ènecarboxylique.